# EUROPÄISCHE PATENTSCHRIFT

(11) **EP 1 976 400 B1**
(45) Veröffentlichungstag und Bekanntmachung des Hinweises auf die Patenterteilung: **20.10.2010**
(21) Anmeldenummer: 06828699.6
(22) Anmeldetag: 19.12.2006
(51) Int. Cl.: A41B 11/00, A41B 11/02

(54) **SOCKE**
SOCK
CHAUSSETTE

(30) Priorität: 27.01.2006 DE 202006001381 U
(43) Veröffentlichungstag der Anmeldung: 08.10.2008
(73) Patentinhaber: X-Technology Swiss GmbH, 8832 Wollerau (CH)
(72) Erfinder: LAMBERTZ, Bodo, W., 8808 Pfäffikon (CH)
(74) Vertreter: Dörner, Lothar
(86) Internationale Anmeldenummer: PCT/DE2006/002269
(87) Internationale Veröffentlichungsnummer: WO 2007/085216

(56) Entgegenhaltungen:
- EP-A- 0 330 588
- DE-U1- 20 119 414
- DE-U1- 20 300 208
- DE-U1- 29 715 762
- FR-A- 2 648 704
- US-A- 5 891 073
- US-A1- 2005 274 045

## Beschreibung

Die Erfindung betrifft eine Socke, insbesondere zum Einsatz bei sportlichen Aktivitäten, mit einem Schaft und einem Fußteil, das einen Zehen- und einen Fersenbereich und einen zwischen Zehen- und Fersenbereich gelegenen Auftrittsbereich aufweist, und die mit einer Bandage versehen ist.

Insbesondere bei sportlichen Aktivitäten sind die menschlichen Füße in der Regel von Socken umgeben. Um eine gute Passform der Socke am Fuß zu erzielen, ist es bekannt, O-Ring-Bandagen an Socken oder Strümpfen vorzuse hen (vgl. bspw. US 5 617 745). Diese sind jedoch parallel zur Längsmittellinie der Socke symmetrisch umlaufend ausgebildet. Aus der DE 297 15 762 U1 ist ein Strumpf bekannt, der eine Socke mit einem symmetrisch verlaufenden Ring beschreibt, der horizontal verläuft. Weiterhin ist aus der EP 0 330 588 A1 ein Strumpf bekannt, bei dem zwei Bandagen vorgesehen sind.

Bei Lauf- und Sprungbewegungen kommt es zu einer erhöhten Belastung des Fußes, insbesondere im Bereich des Sprunggelenkes. Der Fuß hat die natürliche Funktion, zur Dämpfung eines Aufpralls nach innen einzuknicken. Diese Funktion wird Pronation genannt. Nach dem Aufsetzen mit der Außenseite der Sohle verlagert sich die Belastung etwas zur Innenseite, damit das Längsgewölbe des Fußes einsinken und damit einen Teil des Aufpralls absorbieren kann. Der menschliche Fuß kann jedoch unterschiedlich ausgebildet sein. Es wird unterschieden in Normalfuß, Hohl- oder Sichelfuß sowie Senkfuß. Der Normalfuß zeigt ein ausgewogenes Fußgewölbe. Er berührt beim Gehen und Laufen zuerst mit der Außenseite des Rückfußes den Boden. Dann rollt er nach innen ab, um den Aufprall des Fußes aufzufangen und zu dämpfen. Dies wird als natürliche Pronation bezeichnet. Hohl- und Sichelfüße knicken in der Landephase überwiegend nicht nach innen ab und hinterlassen überwiegend im Vor- und Rückfußbereich einen Abdruck. Dies wird als Unterpronation bzw. Supination bezeichnet. Der natürliche Aufprallschutz des Fußes ist bei der Unterpronation stark vermindert. Senkfüße hingegen haben ein sehr niedriges Fußgewölbe und hinterlassen einen kompletten Fußabdruck. Senkfüße knicken nach der Aufsetzphase sehr stark zur Innenseite ab. Dies wird als Überpronation bezeichnet. Außerdem kann der Bewegungsapparat im Bereich der Fußgelenke durch Fehlstellungen der Beine, die üblicher Weise als "O-" bzw. "X-Beine" bezeichnet werden, belastet.

Sowohl Über- als auch Unterpronierer als auch Menschen mit Fehlstellungen der Beine leiden unter dem geringen Maß an natürlicher Dämpfung. Hierdurch wird der Fuß besonders belastet. Um die Sehnen und Bänder des Bewegungsapparates um das Sprunggelenk zu stützen, ist es bekannt, den Fuß zu bandagieren. Hierbei wird eine Bandage horizontal im unteren Bereich des Schienbeins um den Knöchel gewickelt, bevor die Socke über den Fuß gestülpt wird.

Diese Wicklung bietet zum einen nur eine unzureichende Stabilisierung und Stützung des Bewegungsapparates um das Sprunggelenk, zum anderen wird den speziellen Belastungsformen bei Über- bzw. Unterpronation nicht Rechnung getragen. Darüber hinaus trägt die Bandage unter der Socke sehr auf, wodurch der Tragekomfort gemindert ist.

Hier will die Erfindung Abhilfe schaffen. Der Erfindung liegt die Aufgabe zugrunde, eine Socke zu schaffen, die den Bewegungsapparat im Bereich des Sprunggelenkes stützt und dabei speziell für die besonderen Belastungen bei Über- bzw. Unterpronation sowie einer Fehlstellung der Beine ausgelegt ist. Gemäß der Erfindung wird diese Aufgabe dadurch gelöst, dass die Bandage im Bereich des Sprunggelenkes angeordnet ist und aus mindestens einem Steg und mindestens einem Ring besteht, der asymmetrisch auf dem Umfang des Schaftes verläuft.

Mit der Erfindung ist eine Socke, insbesondere für sportliche Aktivitäten geschaffen, die den Bewegungsapparat im Bereich des Sprunggelenkes stützt und speziell für die besonderen Belastungen bei Über- bzw. Unterpronation oder Fehlstellungen der Beine ausgelegt ist. Aus diesem Grund verläuft die Bandage im Bereich des Sprunggelenks, um eine auf den jeweiligen Belastungsfall abgestimmte Stützfunktion erzielen zu können.

Erfindungsgemäss verläuft der Ring auf dem Schaft asymmetrisch. Hierdurch ist eine Stützung insbesondere des Knöchels und somit eine Verbesserung der Stützwirkung hervorgerufen.

In anderer Weiterbildung der Erfindung verläuft der Ring durch den Auftrittsbereich. Hierdurch ist eine Fixierung auch unterhalb des Knöchels des Fußes geschaffen, was eine zusätzliche Unterstützung des Sprunggelenkes hervorruft.

Vorteilhaft verläuft der Steg über den Rist des Fußes. Durch diese Ausbildung besteht die Möglichkeit, in Verbindung mit dem Ring eine besondere Fixierung zu ermöglichen. Auch hier ist bei sportlichen Aktivitäten eine gute Unterstützung hervorgerufen.

Darüber hinaus wird die Aufgabe dadurch gelöst, dass die Bandage aus zwei Ringen besteht, die durch den Auftrittsbereich verlaufen, und dass die Ringe im Auftrittsbereich übereinander gelegt angeordnet sind.. Dadurch ist eine Socke, insbesondere für sportliche Aktivitäten geschaffen, die ebenfalls den Bewegungsapparat im Bereich des Sprunggelenkes stützt und speziell für die besonderen Belastungen bei Über- bzw. Unterpronation oder Fehlstellung der Beine ausgelegt ist. Durch die Verwendung von zwei miteinander kombinierten Ringen ist zudem eine stärkere Bandagierung möglich.

Die ringe sind im Auftrittsbereich übereinander gelegt angeordnet. Es ist dadurch eine im Auftrittsbereich doppellagige Bandagierung möglich, die eine weiter verbesserte Stützfunktion ermöglicht.

Andere Weiterbildungen und Ausgestaltungen der Erfindung sind in den übrigen Unteransprüchen angegeben. Ausführungsbeispiele der Erfindung sind in Zeichnungen dargestellt und werden nachfolgend im Einzelnen beschrieben. Es zeigen:
- Fig. 1a) bis c): eine Socke mit Bandage in der Ansicht der Fußaußen- seite sowie die Ansicht des Sockenpaares von hinten;
- Fig. 2 a) bis c): eine Socke mit Bandage in der Ansicht der Fußaußen- seite sowie die Ansicht des Sockenpaares von hinten in weiterer Ausgestaltung;
- Fig. 3 a) bis c): eine Socke mit Bandage in der Ansicht der Fußaußen- seite sowie die Ansicht des Sockenpaares von hinten in weiterer Ausgestaltung;
- Fig. 4 a) bis c): eine Socke mit Bandage in der Ansicht der Fußaußenseite sowie die Ansicht des Sockenpaares von hinten in weiterer Ausgestaltung;
- Fig. 5 a) und b): eine Socke mit Bandage in der Ansicht der Fußaußenseite sowie der Ansicht von hinten in wei- terer Ausgestaltung;
- Fig. 6 a) und b): eine Socke mit Bandage in der Ansicht der Fußaußenseite sowie der Ansicht von hinten in wei- terer Ausgestaltung und
- Fig. 7 a) und b): eine Socke mit Bandage in der Ansicht der Fußaußenseite sowie der Ansicht von hinten in wei- terer Ausgestaltung;

Die als Ausführungsbeispiel gewählte Socke besteht aus einem Fußteil 1 und einem Schaft 2. Das Fußteil 1 weist einen Zehenbereich 11, einen Fersenbereich 12 und einen zwischen Zehen- und Fersenbereich gelegenen Auftrittsbereich 13 sowie einen Ristbereich 14 auf. Die Bereiche 11,12 und 13 können, wie im Ausführungsbeispiel dargestellt, aus verstärktem Material hergestellt sein. Auch die Verwendung von Materialkombinationen wie beispielsweise Schurwolle mit Elastofaserwerkstoffen ist möglich.

Der Schaft 2 ist an seinem dem Fußteil 1 angewandten Ende mit einem Bund 21 versehen. Im Bereich des Sprunggelenkes ist die Socke mit einer Bandage 3 versehen. Die Bandage 3 ist aus einem elastischen und auch klimaregulierenden Gewebe gebildet. Bevorzugt finden Elastan, Lycra oder andere Materialien unterschiedlicher Dehnbarkeit Anwendung.

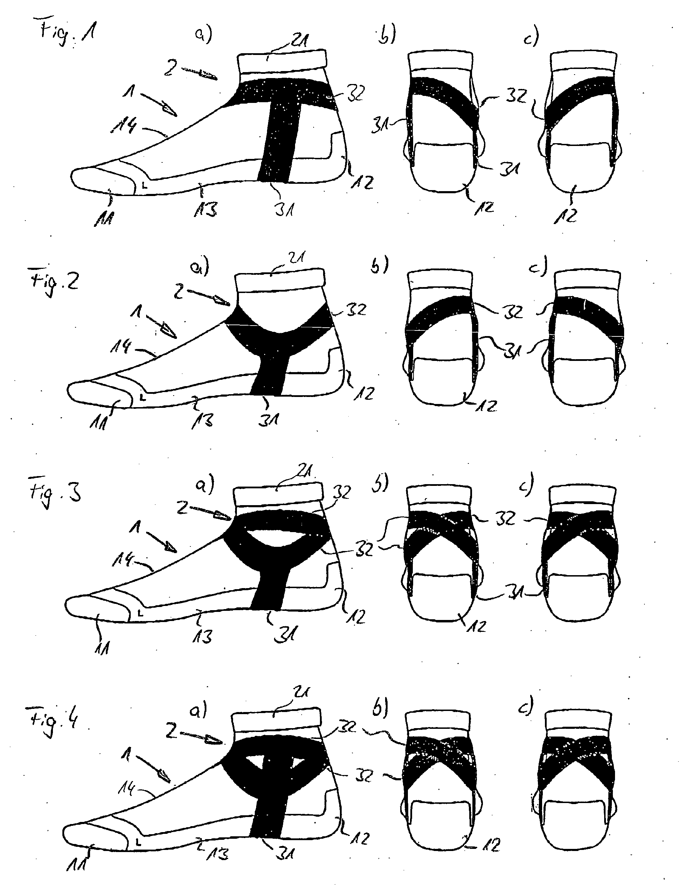

Die Bandage 3 ist durchgängig umlaufend und mit dem die Socke bildenden Gewebe verwoben. Sie ist im Ausführungsbeispiel nach den Figuren 1 bis 6 von einem Steg 31 und einem Ring 32 gebildet. Der Steg 31 verläuft vom Knöchel "K" bis zum Knöchel "K" auf der Außenseite, in dem er den Fuß umrundet.

An das Ende des Steges 31 schließt sich im Bereich der Knöchel der Ring 32 an, der in den Ausführungsbeispielen nach den Figuren 1 bis 6 den Schaft 2 der Socke umschließt und im Wesentlichen auf dessen Umfang verläuft. Der Steg 31 sowie der Ring 32 weisen im Ausführungsbeispiel nach den Figuren 1 bis 6 die gleiche Breite sowie die gleiche Materialstärke auf.

Der Ring 32 ist asymmetrisch angeordnet. Es ergibt sich daraus im Bereich der Ferse eine von der oberen Innen- bzw. Außenseite in Richtung der unteren Innen- bzw. Außenseite schräge Anordnung des Rings 32. Der Ring 32 verläuft wahlweise auf der Fußaußen- oder Fußinnenseite oberhalb des Knöchels "K" (Figuren 1 und 2). In Abwandlung dieser Ausführungsbeispiele ist es auch möglich, eine Kombination von zwei asymmetrisch verlaufenden Ringen 32 vorzustehen (Figuren 3 und 4). Dabei verlaufen sowohl auf der Fußaußen- als auch der Fußinnenseite die Ringe 32 oberhalb und unterhalb des Knöchels "K". Zwischen den oberhalb und unterhalb des Knöchels verlaufenden Ringen ist ein Freiraum hervorgerufen, der eine Kontrollzone bildet. Der Knöchel ist dadurch von den Ringen umgeben. Die Stützfunktion ist hierdurch weiter verbessert.

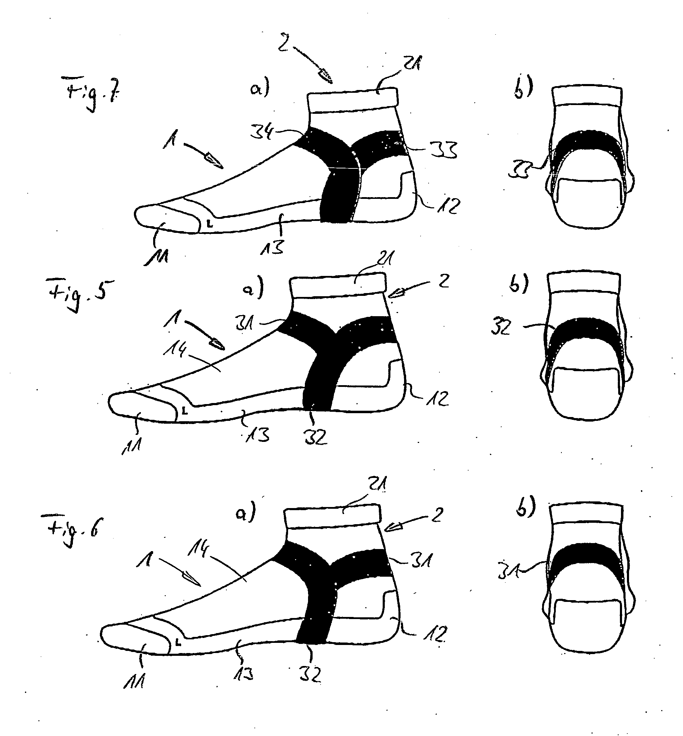

Im Ausführungsbeispiel nach den Figuren 5 und 6 verläuft der Ring 32 durch den Fußauftrittsbereich 13. Im Ausführungsbeispiel nach Figur 5 verläuft der Ring vom Auftrittsbereich 13 über den Bereich der Achillessehne. Der Ring umschließt auf diese Weise die Ferse. Der Steg 31 verläuft im Übergang zwischen Ristbereich 14 und Schaft 2. Die freien Enden des Steges 31 sind an dem Ring 32 angeordnet. Im Ausführungsbeispiel nach Figur 6 ist eine im Vergleich zu dem Ausführungsbeispiel nach Figur 5 umgekehrte Anordnung von Steg 31 und Ring 32 vorgesehen. Hier verläuft der Steg 31 oberhalb des Fersenbereichs 12 im Bereich der Achillessehne; der Ring 32 umschließt die Socke durch den Verlauf vom Auftrittsbereich bis zum Übergang von Ristbereich 14 und Schaft 2. Durch die Ausgestaltungen nach den Ausführungsbeispielen nach den Figuren 5 und 6 ist eine weitere Variierung der Stützfunktion möglich.

Beim Ausführungsbeispiel nach Figur 7 besteht die Socke ebenfalls aus einem Fußteil 1 und einem Schaft 2. Das Fußteil 1 weist einen Zehenbereich 11, einen Fersenbereich 12 und einen zwischen Zehen- und Fersenbereich gelegenen Auftrittsbereich 13 sowie einen Ristbereich 14 auf. Die Bereiche 11, 12 und 13 können, wie im Ausführungsbeispiel dargestellt, aus verstärktem Material hergestellt sein. Ebenso ist die Verwendung von Materialkombinationen möglich. Der Schaft 2 ist an seinem dem Fußteil 1 abgewandten Ende mit einem Bund 21 versehen.

Im Bereich des Sprunggelenkes ist die Socke mit einer Bandage 3 versehen. Die Bandage 3 besteht aus zwei Ringen 33, 34, die durch den Auftrittsbereich 13 verlaufen. Der Ring 33 verläuft vom Auftrittsbereich 13 in den Bereich der Achillessehne und umgibt dadurch die Ferse. Der Ring 34 verläuft vom Auftrittsbereich 13 in den Bereich des Übergangs des Ristbereichs 14 sowie des Schaftes 2. Er verläuft somit im Wesentlichen über den Vorderfuß. Die Ringe 33 und 34 sind im Auftrittsbereich 13 übereinander gelegt angeordnet. Hierdurch ist entweder eine Verdoppelung des Materials im übereinander liegenden Bereich hervorgerufen oder die Ringe sind im Auftrittsbereich miteinander verwoben, sodass eine gleichmäßige aber dennoch festere Struktur geschaffen ist. Hierdurch ist eine noch weitere Erhöhung der Stützfunktion hervorgerufen.

Soweit in der Beschreibung und den Ansprüchen von Socken die Rede ist, beschränkt sich die Erfindung nicht allein auf diese; vielmehr sind unter diesem Begriff auch Strümpfe, Strumpfhosen und dergleichen zu subsumieren, auf die sich die Erfindung ebenfalls bezieht.

## Patentansprüche

1. Socke, insbesondere zum Einsatz bei sportlichen Aktivitäten, mit einem Schaft und einem Fußteil, das einen Zehen- und einen Fersenbereich und einen zwischen Zehen- und Fersenbereich gelegenen Auftrittsbereich aufweist, und die mit einer Bandage versehen ist, **dadurch gekennzeichnet, dass** die Bandage (3) im Bereich des Sprunggelenkes angeordnet ist und aus mindestens einem Steg (31) und mindestens einem Ring (32) besteht, der asymmetrisch auf dem Umfang des Schaftes (2) verläuft.

2. Socke nach Anspruch 1, **dadurch gekennzeichnet, dass** der Ring (32) durch den Auftrittsbereich (13) verläuft.

3. Socke nach Anspruch 2 oder 3, **dadurch gekennzeichnet, dass** der Steg (31) über den Ristbereich (14) verläuft.

4. Socke nach einem der Ansprüche 1 bis 3, **dadurch gekennzeichnet, dass** der Steg (31) oberhalb des Fersenbereichs (12) verläuft.

5. Socke zum Einsatz bei sportlichen Aktivitäten, mit einem Schaft und einem Fußteil, das einen Zehen- und einen Fersenbereich und einen zwischen Zehen- und Fersenbereich gelegenen Auftrittsbereich aufweist, und die mit einer Bandage versehen ist, **dadurch gekennzeichnet, dass** die Bandage (3) aus zwei Ringen (33, 34) besteht, die durch den Auftrittsbereich (13) verlaufen, und dass die Ringe (33, 34) im Auftrittsbereich (13) übereinander gelegt angeordnet sind.

6. Socke nach Anspruch 5, **dadurch gekennzeichnet, dass** der Ring (33) die Ferse umgibt.

7. Socke nach Anspruch 5 oder 6, **dadurch gekennzeichnet, dass** der Ringe (34) über den Ristbereich (14) verläuft.

## Claims

1. Sock, particularly for use in sporting activities, with a leg and a foot portion which foot portion incorporates a toe and a heel area and a tread area disposed between the toe and heel areas and which foot portion is provided with a bandage, **characterised in that** the bandage (3) is disposed in the area of the ankle joint and comprises one or more bands (31) and one or more rings (32) which is or are disposed asymmetrically around the circumference of the leg (2).

2. Sock in accordance with claim 1, **characterised in that** the ring (32) runs through the tread area (13).

3. Sock in accordance with claim 2 or 3, **characterised in that** the band (31) runs across the instep area (14).

4. Sock in accordance with the claims 1 to 3, **characterised in that** the band (31) runs above the heel area (12).

5. Sock, particularly for use in sporting activities, with a leg and a foot portion which incorporates a toe and a heel area and a tread area disposed between the toe and heel areas and which is provided with a bandage, **characterised in that** the bandage (3) consists of two rings (33, 34) which run through the tread area (13) and that the rings (33, 34) are disposed one above the other in the tread area (13).

6. Sock in accordance with claim 5 or 6, **characterised in that** the ring (33) encloses the heel.

7. Sock in accordance with claim 5, **characterised in that** the rings (34) run across the instep area (14).

## Revendications

1. Chaussette à utiliser en particulier dans des activités sportives, avec une partie montante et une partie pied qui présente une zone orteils et une zone talon, ainsi qu'une zone de déroulement du pas située entre la zone orteils et la zone talon, et qui est dotée d'un bandage, **caractérisée en ce que** le bandage (3) est disposé dans la zone de l'articulation astragalo-calcanéenne et se compose d'au moins une nervure (31) et d'au moins un anneau (32) présentant un tracé asymétrique sur le pourtour de la partie montante (2).

2. Chaussette selon la revendication 1, **caractérisée en ce que** le tracé de l'anneau (32) traverse la zone de déroulement du pas (13).

3. Chaussette selon la revendication 2 ou 3, **caractérisée en ce que** le tracé de la nervure (31) passe par le cou-de-pied (14).

4. Chaussette selon l'une des revendications 1 à 3, **caractérisée en ce que** le tracé de la nervure (31) passe au dessus de la zone talon (12).

5. Chaussette à utiliser dans des activités sportives, avec une partie montante et une partie pied qui présente une zone orteils et une zone talon ainsi qu'une zone de déroulement du pas située entre la zone orteils et la zone talon, et qui est dotée d'un bandage, **caractérisée en ce que** le bandage (3) est composé de deux anneaux (33, 34) dont le tracé passe par la zone de déroulement du pas (13), et **en ce que** les anneaux (33, 34) sont agencés superposés dans la zone de déroulement du pas (13).

6. Chaussette selon la revendication 5, **caractérisée en ce que** l'anneau (33) entoure le talon.

7. Chaussette selon la revendication 5 ou 6, **caractérisée en ce que** le tracé de l'anneau (34) passe au dessus de la zone du cou de pied (14).
